(19)

Europäisches
Patentamt
European
Patent Office
Office européen
des brevets

(11)　**EP 3 220 326 A1**

(12) **EUROPEAN PATENT APPLICATION**

(43) Date of publication:
　　20.09.2017　Bulletin 2017/38

(51) Int Cl.:
　　*G06Q 10/06* $^{(2012.01)}$　　　*G06Q 50/22* $^{(2012.01)}$

(21) Application number: **16197525.5**

(22) Date of filing: **07.11.2016**

(84) Designated Contracting States:
　　**AL AT BE BG CH CY CZ DE DK EE ES FI FR GB
　　GR HR HU IE IS IT LI LT LU LV MC MK MT NL NO
　　PL PT RO RS SE SI SK SM TR**
　　Designated Extension States:
　　**BA ME**
　　Designated Validation States:
　　**MA MD**

(30) Priority:　**15.03.2016　KR 20160030938**

(71) Applicant: **Hidea Solutions Co., Ltd.
　　Seoul (KR)**

(72) Inventor: **LEE, SeungYoup
　　Seoul (KR)**

(74) Representative: **De Pablos Riba, Juan Ramon
　　Los Madrazo, 24
　　28014 Madrid (ES)**

(54) **SYSTEM FOR MANAGING OBJECTS FOR CARE WITH DUAL PARAMETER**

(57)　Disclosed is a system for managing an object for care with a dual parameter, including a plurality of detection sensors for generating an activity signal which is a signal system distinguishably representing a deactivation event and an activation event and transmitting the activity signal at every second reference cycle; and a monitoring device configured to receive a signal from the plurality of detection sensors, wherein the monitoring device includes: a receiving unit configured to receive ID information thereof and the activity signal respectively from the plurality of detection sensors at every second reference cycle; and a data generating unit configured to generate mobility information containing movement time information at which the object for care moves from any independent space to another independent space, by parsing the signal system representing a deactivation event and an activation event, included in each of the plurality of transmitted activity signals.

EP 3 220 326 A1

**Description**

TECHNICAL FIELD

The present disclosure relates to a system for monitoring and caring movement, activity or the like of an indoor object for care, and more particularly, to a system for managing an object for care with a dual parameter based on both of a signal system of a sensor distinguishably installed at every independent space partitioned by an indoor barrier or the like and another type of signal system generated through a comparative calculation of the signal system.

BACKGROUND ART

As the society is being modernized fast, the paradigm on family composition is changing, and also along with the increased average life expectancy due to the developed medical science and the improved dietary life, an aging society is coming fast. Due to such a social phenomenon, persons living alone, particularly senior citizens living alone, are remarkably increasing.

In relation to the rapid growth of senior citizens living alone, there is needed intensive social interests and various endeavors for efficient solution, but actually, a local government merely investigates present conditions, or a lifestyle manager merely checks residential conditions or health conditions.

However, such endeavors are mostly dependent on human works and may not be systematically managed. In addition, real-time monitoring and resultant instant follow-up actions may not be performed, and there is a fundamental problem in applying the above measure to all senior citizens living alone or specific persons subject to protection, who need or request management.

There have been proposed several measures to systematically solving the above problem, and such systematic solutions are mostly operated in a way that a sensor is installed indoors to monitor movement of a senior citizen, generate information on a monitoring result and utilize the generated information.

However, in this method, it is checked whether a senior citizen (an object for care) is located at a specific indoor space (a bathroom, a living room, a toilet, a bedroom or the like) just based on a monitoring result obtained by an on/off signal system of an individual sensor, and thus there is a fundamental problem in utilizing this method as a measure for synthetically monitoring overall activity amount, activity scope, moving distance and so on and then generating and utilizing optimized health information dedicated to the object for care by using the monitoring result.

In case of a person suffering from Parkinson's disease, capacity of locomotion is seriously deteriorated due to decreased neurons serving as a component of nerve cells and decreased dopamine serving as a neural transmitter, and in this case, an accurate amount of drug should be taken in accordance with the degree of deteriorated capacity of locomotion in order to manage the nature of disease for a medium and long term.

However, in an existing technique, regardless of the degree of deteriorated capacity of locomotion which may be seriously changed as time goes, medicine is prescribed just based on a brief medical examination or an extremely short observation, and then after a considerable period of time, medicine is prescribed again just based on a brief medical examination or an extremely short observation, repeatedly.

In this case, since quantitative medicine is prescribed and administered based on an instant state of a patient, the patent takes the quantitative medicine successively without considering a following state change of the patient. Thus, the patent is likely to take the medicine too much or too little on the basis of a current state of the patient, which may make the patient worse, in frequent cases.

In order to solve the above problem, there is a method in which a terminal is carried by a patient or attached to a body of the patient so as to monitor a state of the patient, and medicine is prescribed based on the monitoring result. However, in this case, a patient may feel resistance to the terminal, and the patient should make special actions for the terminal, for example carrying or wearing the terminal, periodically charging the terminal or the like. Thus, this method has substantially no effectiveness.

RELATED LITERATURES

Patent Literature

Korean Patent Registration No. 10-1006799 (December 31, 2010)
Korean Unexamined Patent Publication No. 10-2008-0035364 (April 23, 2008)
Korean Unexamined Patent Publication No. 10-2010-0073028 (July 1, 2010)

DISCLOSURE

Technical Problem

The present disclosure is designed to solve the problems of the related art, and therefore the present disclosure is directed to providing a system for managing an object for care with a dual parameter, which may allow an optimized follow-up care service for a senior citizen living alone or a patient by fundamentally solving a burden or resistance of a patient and also monitoring activities of the patient more precisely and successively updating and managing index information to which the monitoring result is reflected.

The present disclosure is also directed to providing enhanced effectiveness for the middle-term or long-term care of a patient in a way that an amount of administrated medicine is differentially controlled according to a state of the patient by reducing the amount of administrated medicine or elongating an administration interval when a behavior characteristic of the patient becomes worse and by increasing the amount of administrated medicine or shortening an administration interval when a behavior characteristic of the patient becomes better.

Technical Solution

In one aspect of the present disclosure, there is provided a system for managing an object for care with a dual parameter, comprising: a plurality of detection sensors installed at every independent space partitioned by barriers to detect an object for care in a detection area at every first reference cycle, the plurality of detection sensors generating an activity signal which is a signal system distinguishably representing a.deactivation event and an activation event and transmitting the activity signal at every second reference cycle; and a monitoring device configured to receive a signal from the plurality of detection sensors, wherein the monitoring device includes: a receiving unit configured to receive ID information thereof and the activity signal respectively from the plurality of detection sensors at every second reference cycle; and a data generating unit configured to generate mobility information containing movement time information at which the object for care moves from any independent space to another independent space, by parsing the signal system representing a deactivation event and an activation event, included in each of the plurality of transmitted activity signals.

Here, the activity signal may be a signal system distinguishably representing a deactivation event at which any one of a temperature change equal to or greater than a reference temperature difference and a speed of the object for care equal to or greater than a reference speed is detected and an activation event at which both of a temperature change equal to or greater than a reference temperature difference and a speed of the object for care equal to or greater than a reference speed are detected.

Also, the data generating unit may generate the mobility information by comparing two or more activity signals containing a signal system changing from the activation event to the deactivation event or changing from the deactivation event to the activation event.

In addition, the monitoring device may further include a location information storing unit configured to store location information of the plurality of detection sensors, and the data generating unit may further generate moving direction information or moving distance information at which the object for care moves from any independent space to another independent space, by using the ID information or the location information of the plurality of detection sensors, and generate mobility information further containing the generated moving direction information or moving distance information.

In an embodiment, the monitoring device may further include a location information storing unit configured to store point location information which is location information of a passage between the independent spaces, and the data generating unit may further generate moving direction information or moving distance information at which the object for care moves from any independent space to another independent space, by using the point location information, and generate mobility information further containing the generated moving direction information or moving distance information.

Preferably, the system for managing an object for care with a dual parameter according to the present disclosure may further include a reference index generating unit configured to update and generate reference index information about activity of the object for care by using statistical data with respect to mobility information generated from a first stage to $n^{th}$ stage, when $n^{th}$ mobility information (n is a natural number of 2 or above) is generated.

In addition, the system for managing an object for care with a dual parameter according to the present disclosure may further include a management control unit configured to calculate a difference between the reference index information and current index information generated using statistical data of mobility information at a current stage or mobility information generated at $m^{th}$ stage (m is a natural number of 2 or above) before at least one stage than the current stage, and generate a differential management control signal according to a calculation result.

Further, the system for managing an object for care with a dual parameter according to the present disclosure may further include a dispenser configured to control a discharge interval or amount of medicine which is stored therein and discharged out according to a transmitted signal, and the management control unit may transmit the management control signal to the dispenser so that medicine is discharged from the dispenser at a differential interval or by a differential amount.

In addition, the system for managing an object for care with a dual parameter according to the present disclosure may further include an electric or electronic device controlled to operate according to a transmitted signal, and the management control unit may transmit the management control signal to the electric or electronic device so that the electric or electronic device differentially turns on or off.

Advantageous Effects

The system for managing an object for care with a dual parameter according to the present disclosure does not demand any special action to a patient, and thus the system for analyzing and monitoring a behavior characteristic of an object for care (a patient or the like) may be implemented in a user-oriented way.

In addition, according to an embodiment of the present disclosure, a dual parameter organically combined may be introduced, and an object for care may be monitored more precisely and more accurately based on the dual parameter.

Further, according to an embodiment of the present disclosure, a differential management signal system may be generated based on a current state of a patient which is varying continuously, and an amount of administered medicine or an administration interval may be automatically controlled accordingly, which may implement a medium-term and long-term follow-up care system optimized for a current state of a patient.

DESCRIPTION OF DRAWINGS

Fig. 1 is a diagram showing a managing system according to an embodiment of the present disclosure and relevant components as a whole.

Fig. 2 is a block diagram showing a detailed configuration of the managing system according to an embodiment of the present disclosure.

Fig. 3 is a flowchart for illustrating a process of generating an activity signal system according to an embodiment of the present disclosure.

Fig. 4 is a diagram for illustrating an activity signal system according to an embodiment of the present disclosure.

Fig. 5 is a flowchart for illustrating a process of generating mobility information according to an embodiment of the present disclosure.

Fig. 6 is a diagram for illustrating mobility information according to an embodiment of the present disclosure.

Fig. 7 is a flowchart for illustrating a process of generating index information and a process of generating a management control signal according to an embodiment of the present disclosure.

BEST MODE

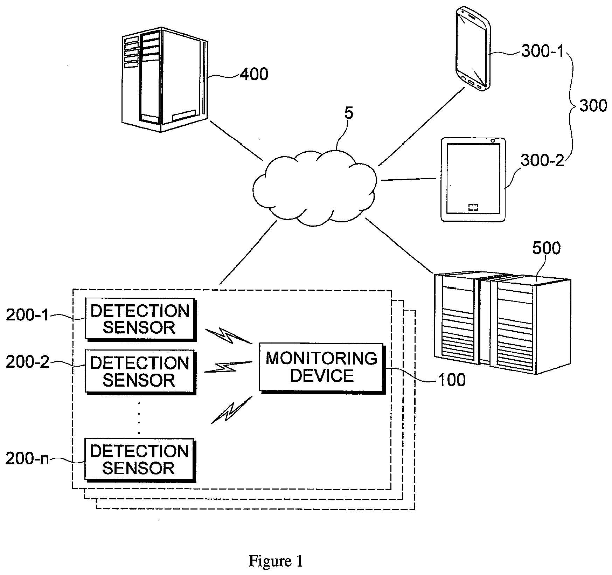

Fig. 1 is a diagram showing a system 1000 for managing an object for care with a dual parameter (hereinafter, also referred to as a 'managing system') according to an embodiment of the present disclosure as a whole.

As shown in Fig. 1, the managing system 1000 of the present disclosure includes a plurality of detection sensors 200-1, 200-2, 200-n provided indoors and a monitoring device 100 provided indoors.

The monitoring device 100 of the present disclosure may be communicatively connected to a service server 400 of the present disclosure through a gateway, a communication network 5 or the like. As explained later, several processes performed at the monitoring device 100 of the present disclosure may be implemented without any physical limitation, and thus these processes may also be performed at the service server 400 of the present disclosure which is communicated with a plurality of monitoring devices 100.

In addition, if any dangerous factor is detected or any cautious situation occurs by means of continuous monitoring of a signal system detected indoors and statistic calculation, the monitoring device 100 or the service server 400 of the present disclosure may make a call or send a text message to an object for care or a protector terminal 300-1, 300-n of the object for care, and the monitoring device 100 or the service server 400 of the present disclosure may also be communicatively connected to an agent server 500 of a police station, a fire station, a medical institution or the like to share necessary data.

The communication network 5 performs data communication among the monitoring device 100, the service server 400, the agent server 500 and the protector terminal 300 of the present disclosure and may have any of wired, wireless and wire/wireless combination types. The communication network 5 may also be implemented with various protocols and methods such as LAN, CDMA, WCDMA, LTE, WiFi or the like. In addition, the communication between the plurality of detection sensors 200 installed indoors and the monitoring device 100 are performed by means of ZigBee communication in consideration of power consumption, size of transmitted data, communication coverage or the like.

Hereinafter, the overall configuration and processing of the managing system 1000 according to the present disclosure will be described in detail with reference to the accompanying drawings.

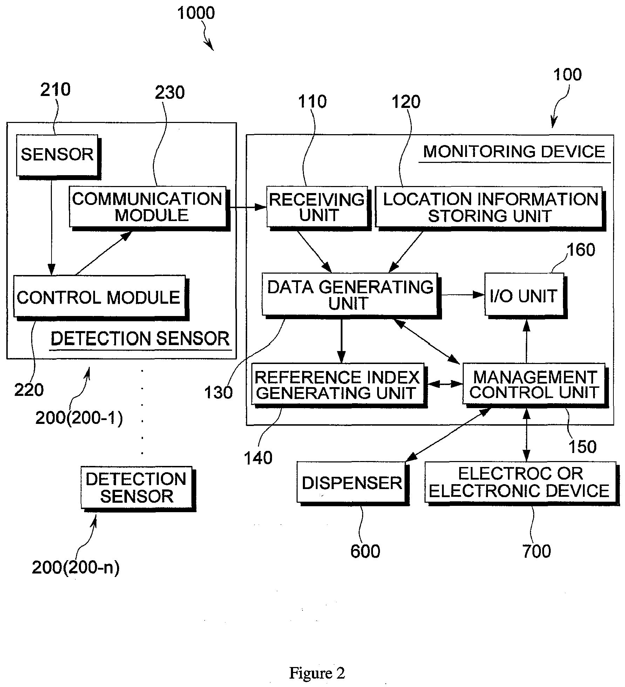

As shown in Fig. 2, the managing system 1000 of the present disclosure includes a plurality of detection sensors 200 and a monitoring device 100. The detection sensor 200 may include a sensor 210, a control module 220 and a communication module 230, and the monitoring device 100 may include a receiving unit 110, a location information storing unit 120, a data generating unit 130, a reference index generating unit 140, a management control unit 150, and an I/O unit 160.

Prior to explaining the present disclosure in detail, it should be understood that components of the detection sensor 200 and the monitoring device 100 according to the present disclosure as depicted in Fig. 2 are not physically distinguished but logically distinguished.

In other words, each component corresponds to a logic component for implementing a technical feature of the present disclosure, and thus even though any components are integrated or divided, it should be interpreted as falling within the scope of the present disclosure as long as the function of the logic component of the present disclosure is accomplished. Also, any components having identical or similar functions should be interpreted as falling within the scope of the present disclosure regardless whether their terms are identical or not.

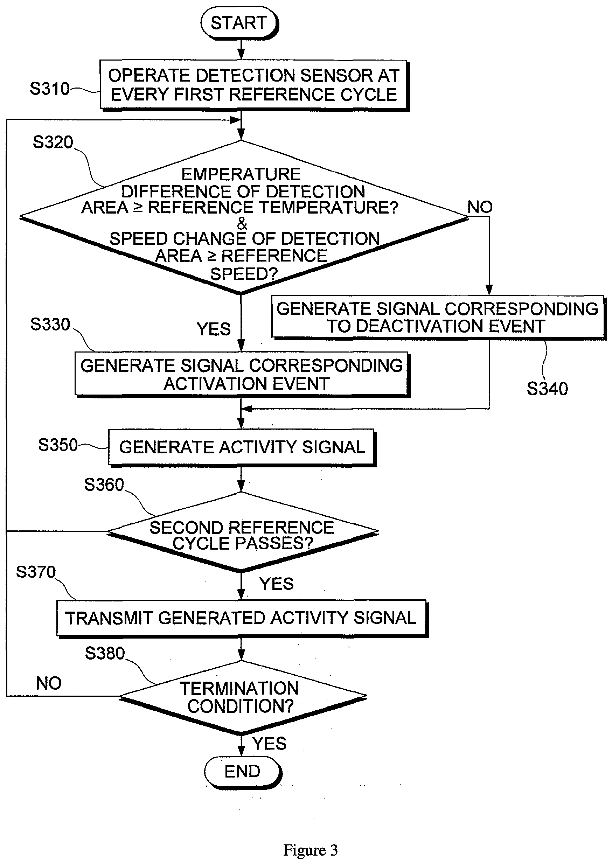

First, detailed configuration and processing of the detection sensor 200 according to the present disclosure will be described with reference to Figs. 2 and 3.

The detection sensor 200 of the present disclosure includes a sensor 210 for sensing an object for care in a detection area and a control module 220 for processing data of the signal received from the sensor 210 according to set transmission interval and protocol and controlling the communication module 230 to transmit the generated signal to the monitoring device 100 of the present disclosure.

The detection sensor 200 of the present disclosure is installed at every independent space partitioned by barriers. The barrier means a physical block for preventing an internal signal from being transmitted outwards, and when the detection sensor 200 is installed indoors, walls for partitioning physical spaces such as a first room a second room, a living room and a bathroom may correspond to the barriers.

In the present disclosure, the detection sensor 200 is installed at each independent space, but it is also possible to install a plurality of detection sensors 200 in a single independent space in order to enhance detection efficiency and precision of location information for moving distance information. Thus, the number of detection sensors 200 installed in each independent space is not specially limited.

The sensor 210 may adopt various kinds of sensors using infrared heat detection, detection of reflected infrared wavelength change, UWB, visible rays or the like. Since the sensor is likely to be installed indoors, a human body emitting heat from itself becomes a main object for care, the sensor may be an infrared sensor which may detect a change and amount of reflected infrared wavelength, in order to effectively overcome interference with light emitting from a lamp and also effectively detect a temperature difference and a speed difference as explained below.

The detection sensor 200 of the present disclosure detects an object for care in a detection area at every first reference cycle, which may be set variably (S310). Here, among movements of the object for care, only an effective movement, namely only a movement regarded as an activity or behavior characteristic of the object for care, may be detected so that the detection data serving as raw data for various following processes of the present disclosure, explained later, have reliability and also further improve the precision of other following processes.

For this, in the present disclosure, it is designed to detect that an effective movement occurs only when all conditions defined in the following equations are satisfied. In the present disclosure, the 'occurrence of an effective movement' is called an 'activation event' for convenient explanation and understanding, and a case when conditions defined in the following equations are not entirely satisfied, namely the 'occurrence of an ineffective movement' is called a 'deactivation event'.

Equation 1

$$\Delta T \geq \Delta T_r$$

$$V \geq V_r$$

In Equation 1, $\Delta T$ represents a temperature difference detected in the detection area, $\Delta T$ represents reference temperature difference information which may be set variably, V represents speed information detected in the detection area, and Vr represents reference speed information which may be set variably.

As described above, it is determined whether the temperature difference information detected in the detection area is equal to or greater than reference temperature difference information and whether the detected speed information is equal to or greater than reference speed information (S320). If both of two conditions are satisfied, a signal representing an activation event is generated (S330), and if any one of two conditions is not satisfied, a signal representing a deactivation event is generated (S340).

As described above, the detection sensor 200 of the present disclosure generates a signal system distinguishably representing an activation event and a deactivation event at every sampling cycle (S350). In the present disclosure, this signal system is called an activity signal.

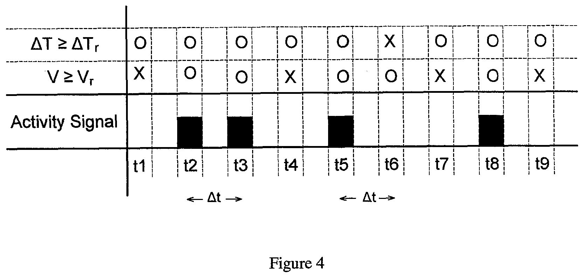

Fig. 4 is a diagram for illustrating a process of generating an activity signal as described above.

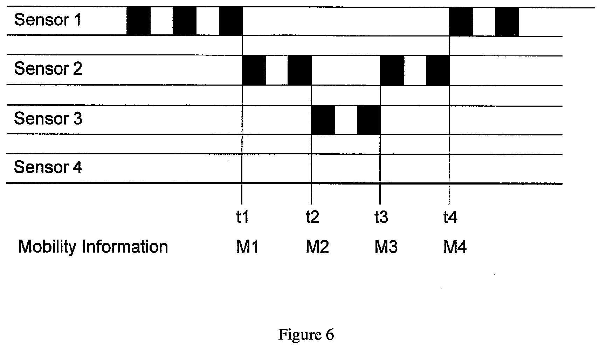

As shown in Fig. 4, at times t1, t4, t6, t7, t9, two conditions as described above are not entirely satisfied, and thus a signal system corresponding to the deactivation event is generated. Also, at times t2, t3, t5, t8, two conditions are entirely satisfied, and thus a signal system corresponding to the activation event is generated. In Fig. 6, $\Delta t$ corresponds to a first reference cycle.

The signal systems corresponding to an activation event and a deactivation event are representatively data signals of "1" and "0", and various kinds of signal systems may be used as long as the signal systems are capable of distinguishing each event.

In this way, the detection sensor 200 of the present disclosure generates an activity signal in relation to the detection of the object for care at every first reference cycle during a set second reference cycle, and if the second reference cycle passes (S360), the detection sensor 200 of the present disclosure transmits a series of activity signal systems generated until now to the monitoring device 100 of the present disclosure along with ID information thereof (S370).

As long as a specific termination condition (an artificial termination signal, a power-off or the like) is not made (S380), the above process is performed circularly and repeatedly.

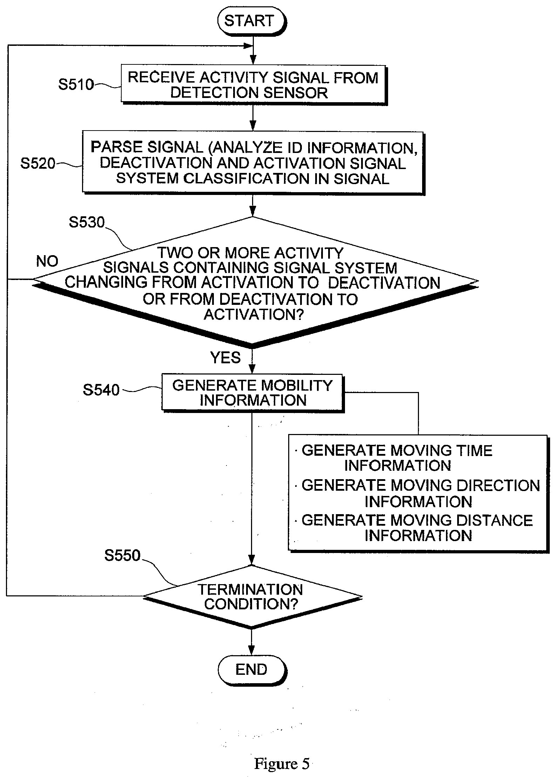

Hereinafter, detailed configuration and processing of the monitoring device 100 according to the present disclosure will be described with reference to Figs. 5 to 7.

Fig. 5 is a flowchart for illustrating a process of generating mobility information, which may be performed at the monitoring device 100 of the present disclosure or the service server 400 of the present disclosure, according to an embodiment.

The receiving unit 110 of the monitoring device 100 according to the present disclosure receives its ID information and the activity signal at every second reference cycle from each of the plurality of detection sensors 200 installed indoors (S510).

If the activity signal is received, the data generating unit 130 of the present disclosure parses the received signal to distinguish the ID information, the signal representing a deactivation event, the signal system representing an activation event and so on of the received signal (S520).

If the signal system is completely parsed, the data generating unit 130 of the present disclosure determines whether the signal representing an activation event and the signal representing a deactivation event correspond to each other, and generates mobility information which contains movement time information showing that the object for care moves from any independent space to another independent space, based on whether the activity signals correspond to each other (S440).

In the present disclosure, only when it is checked that the object for care moves from any independent space to another independent space by analyzing the activity signal system, it is regarded that the object for care effectively moves, and then information representing a behavior characteristic of the object for care is effectively generated. The information of the present disclosure generated as above is called 'mobility information' for convenient explanation and understanding.

As described above, in the present disclosure, the activity signal' showing that the object for care makes an effective movement and the 'mobility information' generated based on the 'activity signal' and showing that an effective movement is made are generated dually, thereby generating an index about a behavior characteristic of the object for care more precisely and more effectively.

Fig. 6 is a diagram for illustrating mobility information according to an embodiment of the present disclosure.

Fig. 6 shows that activity signals are received from four detection sensors 200. As shown in Fig. 6, at a time t1, in case of Sensor 1 (a first detection sensor), a signal corresponding to an activation event is changed into a signal corresponding to a deactivation event, and at the identical time t1, in case of Sensor 2 (a second detection sensor), a signal corresponding to a deactivation event is changed into a signal corresponding to an activation event (S530).

By preparing for the time when an activation event or a deactivation event is changed, accurate time information when the object for care moves from any independent space to another independent space may be generated. Thus, the data generating unit 130 of the present disclosure generates mobility information containing time information at the times t1, t2, t3 and t4 as depicted in Fig. 6, namely time information showing when the object for care moves from space to space (S540). The process of generating mobility information is also performed repeatedly and circularly unless a specific terminal condition is satisfied (S550).

The location information storing unit 120 of the monitoring device 100 according to the present disclosure stores location information of the plurality of detection sensors 200, and information about a moving direction of the object for care may be generated using the ID information of each detection sensor 200 changing from an activation event signal to a deactivation event signal or changing from a deactivation event signal to an activation event signal or the location information corresponding thereto.

Further, if time when next mobility information is generated and relevant information is analyzed, information about a moving distance of the object for care for a specific time may also be generated.

Since movement between indoor independent spaces is made through a door, distance information or location information of a first door (between a room and a living room) and a second door (between the living room and a bathroom) are stored in the location information storing unit 120 of the present disclosure or the like as a DB, and then a moving distance may be accurately extracted by checking from which space and to which space the movement is made, on the basis of the mobility information. For this, the location information storing unit 120 of the present disclosure may store point location information which is location information of a structure or place of a door through which a movement is made between independent spaces.

In other words, the data generating unit 130 of the present disclosure may accurately calculate a moving distance of the object for care by using ID information of the detection sensors 200 whose signal system changes from an activation event to a deactivation event at a specific time (or, from a deactivation event to an activation event), ID information of the detection sensors 200 whose signal system changes from an activation event to a deactivation event at another specific time (or, from a deactivation event to an activation event), and the point location information.

In addition, a difference between a time when first mobility information (M1) representing a movement from a first space (a room) to a second space (a living room) is generated and a time when mobility information (M2) representing a movement from a second space (the living room) to a third space (a bathroom) may be accurately calculated, and thus accurate speed information of the object for care may also be calculated based on the moving distance information. Through the above method, movement time information, moving distance information and speed information of the object for care are accurately recorded in the mobility information of the present disclosure.

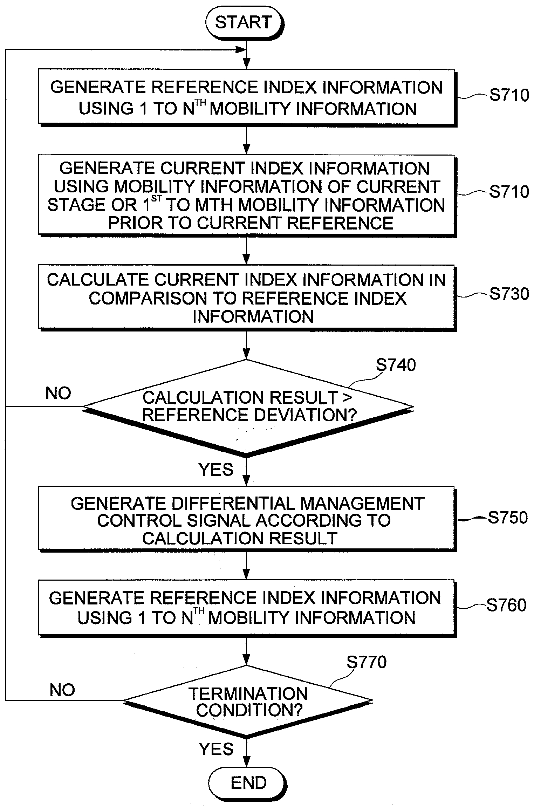

Fig. 7 is a flowchart for illustrating a process of generating index information and a process of generating a management control signal according to an embodiment of the present disclosure.

As shown in Fig. 7, if mobility information of a current stage (namely, $n^{th}$ mobility information (n is a natural number of 2 or above) is generated, the reference index generating unit 140 of the present disclosure continuously updates and generates reference index information about a behavior characteristic or activity of the object for care by statistically calculating mobility information (moving time information, moving distance information, speed information or the like) generated before the mobility information of the current stage is generated (first to n-$1^{th}$ mobility information) (S710).

The reference index information serves as a reference for determining a behavior characteristic, activity particularity, deviation or the like of the object for care at a current stage, and mobility information of the current stage and next stages are continuously reflected thereon to update the mobility information so that the mobility information may be utilized as more effective reference information.

The statistic calculation for generating the reference index information may be averaging of entire mobility information, but a weight mean may also be applied depending on age, sex, medical history, symptom or the like of the object for care. Other statistic methods or calculations may also be applied.

The management control unit 150 of the present disclosure generates current index information by using mobility information of a current stage or statistic data of mobility information of several former stages prior to the current stage (S720), and compares the current index information with the reference index information described above to calculate a difference between them (S730).

The current index information may be composed of only mobility information generated at a current stage or may also be composed using static data of mobility information generated from first to $m^{th}$ stages (m is a natural number of 2 or above) prior to the current stage in order to reflect recent motion or movement.

Through this calculation, features of a current behavior pattern or tendency of the object for care may be checked in comparison to a behavior pattern or tendency which serves as a reference. If a difference or deviation between the current index information and the reference index information is smaller than a reference deviation (S740), it may be regarded that there is no serious meaningful change in the behavior pattern in comparison to an ordinary pattern.

However, if a difference between the current index information and the reference index information is greater than the reference deviation as a result of calculation (S740), a differential management control signal is generated according to the calculation result (S750).

The differential management control signal may be utilized in various ways. For example, if management control signals are classified into symptom, caution, warning, emergency or the like, a guidance message conforming to each classification may be sent to a terminal of the object for care or a protector terminal 300 thereof, or the corresponding content may be sent through a telephone call.

In this regard, the present disclosure may be configured to associate with a dispenser 600 having a function of discharging a medicine stored therein so that the medicine may be administrated to a patient suffering from Parkinson's disease by an accurate amount and at an accurate interval.

The dispenser 600 stores a medicine therein and allows the stored medicine to be discharged by opening a discharge hole by means of user manipulation.

An electric or electronic device 700 or the like is loaded in the dispenser 600 to control an opening/closing cycle and time of the discharge hole or an amount of discharged medicine according to an external signal, and the management control unit 150 of the present disclosure transmits a differential management control signal according to the degree of deviation to the dispenser 600 so that an opening/closing cycle and time of the discharge hole of the dispenser 600 may be controlled according to the received management control signal, thereby guiding an accurate amount of medicine conforming to a current behavior characteristic or state of the patient to be administrated.

In addition, necessary parts may be loaded on the corresponding electric or electronic device 700 to be in association with an audiovisual medium such as a TV and an audio, and if the management control unit 150 of the monitoring device 100 according to the present disclosure transmits a management control signal, the corresponding electric or electronic device 700 may be controlled by the transmitted management control signal to turn on/off or turn up the volume.

Depending on embodiments, the present disclosure may be associated with an electric or electronic exercise machine such as a physical massager, an electric stimulator or the like, used by the object for care (a patient or the like), so that while the object for care is exercising, the intensity of exercise may be adjusted in a state where the object for care does not recognize.

The monitoring device 100 described above may also be configured to just store or collect data received from the detection sensors 200 and transmit the received data to the service server 400 of the present disclosure so that the calculating, comparing and determining processes of the monitoring device 100 in the former embodiment may be performed by the service server 400 and the process results are fed back to the monitoring device 100.

Reference Symbols

1000: system for managing an object for care

| | |
|---|---|
| 100: monitoring device | 110: receiving unit |
| 120: location information storing unit | 130: data generating unit |
| 140: reference index generating unit | 150: management control unit |
| 160: I/O unit | |
| 200: detection sensor | 210: sensor |
| 220: control module | 230: communication module |
| 300: protector (patient) terminal | 400: service server |
| 500: agent server | |

**Claims**

1. A system for managing an object for care with a dual parameter, comprising:

a plurality of detection sensors installed at every independent space partitioned by barriers to detect an object for care in a detection area at every first reference cycle, the plurality of detection sensors generating an activity signal which is a signal system distinguishably representing a deactivation event and an activation event and transmitting the activity signal at every second reference cycle; and
a monitoring device configured to receive a signal from the plurality of detection sensors,

wherein the monitoring device includes:

a receiving unit configured to receive ID information thereof and the activity signal respectively from the plurality of detection sensors at every second reference cycle; and
a data generating unit configured to generate mobility information containing movement time information at which the object for care moves from any independent space to another independent space, by parsing the signal system representing a deactivation event and an activation event, included in each of the plurality of transmitted activity signals.

2. The system for managing an object for care with a dual parameter according to claim 1,
wherein the activity signal is a signal system distinguishably representing a deactivation event at which any one of a temperature change equal to or greater than a reference temperature difference and a speed of the object for care equal to or greater than a reference speed is detected and an activation event at which both of a temperature change equal to or greater than a reference temperature difference and a speed of the object for care equal to or greater than a reference speed are detected.

3. The system for managing an object for care with a dual parameter according to claim 2,
wherein the data generating unit generates the mobility information by comparing two or more activity signals containing a signal system changing from the activation event to the deactivation event or changing from the deactivation event to the activation event.

4. The system for managing an object for care with a dual parameter according to claim 3,
wherein the monitoring device further includes a location information storing unit configured to store location information of the plurality of detection sensors, and
wherein the data generating unit further generates moving direction information or moving distance information at which the object for care moves from any independent space to another independent space, by using the ID information or the location information of the plurality of detection sensors, and generates mobility information further containing the generated moving direction information or moving distance information.

5. The system for managing an object for care with a dual parameter according to claim 3,
wherein the monitoring device further includes a location information storing unit configured to store point location information which is location information of a passage between the independent spaces, and
wherein the data generating unit further generates moving direction information or moving distance information at which the object for care moves from any independent space to another independent space, by using the point location information, and generates mobility information further containing the generated moving direction information or moving distance information.

6. The system for managing an object for care with a dual parameter according to claim 4 or 5, further comprising:

a reference index generating unit configured to update and generate reference index information about activity of the object for care by using statistical data with respect to mobility information generated from a first stage to $n^{th}$ stage, when $n^{th}$ mobility information (n is a natural number of 2 or above) is generated.

7. The system for managing an object for care with a dual parameter according to claim 6, further comprising:

a management control unit configured to calculate a difference between the reference index information and current index information generated using statistical data of mobility information at a current stage or mobility information generated at $m^{th}$ stage (m is a natural number of 2 or above) before at least one stage than the current stage, and generate a differential management control signal according to a calculation result.

8. The system for managing an object for care with a dual parameter according to claim 7, further comprising:

a dispenser configured to control a discharge interval or amount of medicine which is stored therein and discharged out according to a transmitted signal,
wherein the management control unit transmits the management control signal to the dispenser so that medicine is discharged from the dispenser at a differential interval or by a differential amount.

9. The system for managing an object for care with a dual parameter according to claim 7, further comprising:

an electric or electronic device controlled to operate according to a transmitted signal,
wherein the management control unit transmits the management control signal to the electric or electronic device so that the electric or electronic device differentially turns on or off.

Figure 1

1000

100

210    230

110    120

SENSOR

MONITORING DEVICE

COMMUNICATION
MODULE

RECEIVING
UNIT

LOCATION INFORMATION
STORING UNIT

160

CONTROL MODULE

DATA GENERATING
UNIT

I/O UNIT

DETECTION SENSOR

220

REFERENCE INDEX
GENERATING UNIT

MANAGEMENT
CONTROL UNIT

200(200-1)

130

140

150

DETECTION
SENSOR

DISPENSER

ELECTROC OR
ELECTRONIC DEVICE

200(200-n)

600    700

Figure 2

START

S310 — OPERATE DETECTION SENSOR AT EVERY FIRST REFERENCE CYCLE

S320 — EMPERATURE DIFFERENCE OF DETECTION AREA ≥ REFERENCE TEMPERATURE? & SPEED CHANGE OF DETECTION AREA ≥ REFERENCE SPEED?

NO

GENERATE SIGNAL CORRESPONDING TO DEACTIVATION EVENT

S340

YES

S330 — GENERATE SIGNAL CORRESPONDING ACTIVATION EVENT

S350 — GENERATE ACTIVITY SIGNAL

S360 — SECOND REFERENCE CYCLE PASSES?

YES

S370 — TRANSMIT GENERATED ACTIVITY SIGNAL

S380 — TERMINATION CONDITION?

NO

YES

END

Figure 3

| | t1 | t2 | t3 | t4 | t5 | t6 | t7 | t8 | t9 |
|---|---|---|---|---|---|---|---|---|---|
| $\Delta T \geq \Delta T_r$ | O | O | O | O | O | X | O | O | O |
| $V \geq V_r$ | X | O | O | X | O | O | X | O | X |
| Activity Signal | | ■ | ■ | | ■ | | | ■ | |

← Δt →     ← Δt →

Figure 4

START

S510 — RECEIVE ACTIVITY SIGNAL FROM DETECTION SENSOR

S520 — PARSE SIGNAL (ANALYZE ID INFORMATION, DEACTIVATION AND ACTIVATION SIGNAL SYSTEM CLASSIFICATION IN SIGNAL

S530 — TWO OR MORE ACTIVITY SIGNALS CONTAINING SIGNAL SYSTEM CHANGING FROM ACTIVATION TO DEACTIVATION OR FROM DEACTIVATION TO ACTIVATION?

NO

YES

S540 — GENERATE MOBILITY INFORMATION

· GENERATE MOVING TIME INFORMATION
· GENERATE MOVING DIRECTION INFORMATION
· GENERATE MOVING DISTANCE INFORMATION

S550 — TERMINATION CONDITION?

END

Figure 5

Figure 6

EP 3 220 326 A1

START

GENERATE REFERENCE INDEX INFORMATION
USING 1 TO $N^{TH}$ MOBILITY INFORMATION — S710

GENERATE CURRENT INDEX INFORMATION
USING MOBILITY INFORMATION OF CURRENT
STAGE OR $1^{ST}$ TO MTH MOBILITY INFORMATION
PRIOR TO CURRENT REFERENCE — S710

CALCULATE CURRENT INDEX INFORMATION IN
COMPARISON TO REFERENCE INDEX
INFORMATION — S730

S740

NO — CALCULATION RESULT >
REFERENCE DEVIATION?

YES

GENERATE DIFFERENTIAL MANAGEMENT
CONTROL SIGNAL ACCORDING TO
CALCULATION RESULT — S750

GENERATE REFERENCE INDEX INFORMATION
USING 1 TO $N^{TH}$ MOBILITY INFORMATION — S760

S770

NO — TERMINATION
CONDITION?

YES

END

Europäisches
Patentamt

European
Patent Office

Office européen
des brevets

# EUROPEAN SEARCH REPORT

Application Number

EP 16 19 7525

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 421 896 A2 (SEIKO INSTR INC [JP]) 26 May 2004 (2004-05-26) * the whole document * ----- | 1-9 | INV. G06Q10/06 G06Q50/22 |
| X | US 2010/161354 A1 (LIM JOON HO [KR] ET AL) 24 June 2010 (2010-06-24) * paragraph [0028]; figure 1 * ----- | 1-9 | |
| X | KR 2015 0137778 A (YANG BUM SUK [KR]; KYUNG KYU HO [KR]) 9 December 2015 (2015-12-09) * the whole document * * paragraph [0010] - paragraph [0013] * ----- | 1-9 | |
| X | US 2008/084296 A1 (KUTZIK DAVID [US] ET AL) 10 April 2008 (2008-04-10) * paragraph [0045] - paragraph [0055] * ----- | 1-9 | |

TECHNICAL FIELDS
SEARCHED      (IPC)

G06Q

The present search report has been drawn up for all claims

| Place of search | Date of completion of the search | Examiner |
|---|---|---|
| Munich | 30 November 2016 | Anastasov, Yuliyan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................................................
& : member of the same patent family, corresponding
    document

EPO FORM 1503 03.82 (P04C01)

**EP 3 220 326 A1**

## ANNEX TO THE EUROPEAN SEARCH REPORT
## ON EUROPEAN PATENT APPLICATION NO.

EP 16 19 7525

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2016

| Patent document cited in search report | | Publication date | Patent family member(s) | | Publication date |
|---|---|---|---|---|---|
| EP 1421896 | A2 | 26-05-2004 | CA | 2450051 A1 | 19-05-2004 |
| | | | EP | 1421896 A2 | 26-05-2004 |
| | | | JP | 2004216125 A | 05-08-2004 |
| | | | US | 2004113771 A1 | 17-06-2004 |
| US 2010161354 | A1 | 24-06-2010 | KR | 20100073028 A | 01-07-2010 |
| | | | US | 2010161354 A1 | 24-06-2010 |
| KR 20150137778 | A | 09-12-2015 | NONE | | |
| US 2008084296 | A1 | 10-04-2008 | US | 2008084296 A1 | 10-04-2008 |
| | | | WO | 2008156956 A1 | 24-12-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101006799
- KR 1020080035364
- KR 1020100073028